# EUROPEAN PATENT APPLICATION

(11) **EP 3 680 227 A1**
(43) Date of publication of application: **15.07.2020**
(21) Application number: 18853433.3
(22) Date of filing: 07.08.2018
(51) Int. Cl.: C07C 51/08, C07C 59/86

(54) **METHOD FOR PREPARING SUBSTITUTED PHENYLACETIC ACID DERIVATIVE**

(30) Priority: 07.09.2017 CN 201710800788
(71) Applicant: Jiangsu Ruike Medical Science And Technology Co., Ltd., Jiangsu 224100 (CN)
(72) Inventor: LI, Jieping, Taizhou Zhejiang 318000 (CN); GAO, Zhaobo, Taizhou Zhejiang 318000 (CN); CHAI, Bing, Taizhou Zhejiang 318000 (CN); ZHENG, Hui, Taizhou Zhejiang 318000 (CN); LIU, Shengmin, Taizhou Zhejiang 318000 (CN); LIU, Aqing, Taizhou Zhejiang 318000 (CN); GUO, Bibao, Taizhou Zhejiang 318000 (CN); ZHENG, Juncheng, Taizhou Zhejiang 318000 (CN); WANG, Changfa, Taizhou Zhejiang 318000 (CN); LU, Weiwei, Taizhou Zhejiang 318000 (CN)
(74) Representative: Grünecker Patent- und Rechtsanwälte PartG mbB
(86) International application number: PCT/CN2018/099121
(87) International publication number: WO 2019/047654

(57) **Abstract**

The invention belongs to the pharmaceutical manufacturing field, which relates to a novel process for the preparation of substituted phenylacetic acids derivatives, especially relates to the preparation of 2-(4-(2-oxocyclopentyl)phenyl)propanoic acid. The process for the preparation of the precursor form of loxoprofen which use 1,4-di-halobenzylcompounds or disubstituted benzyl compounds as starting material, is through the substitution reaction of cyclopentanone groups or its precursor compounds.

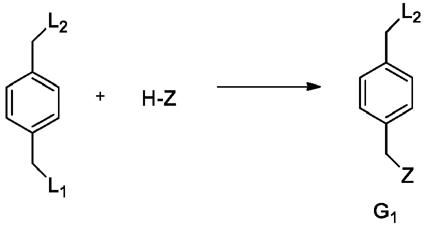

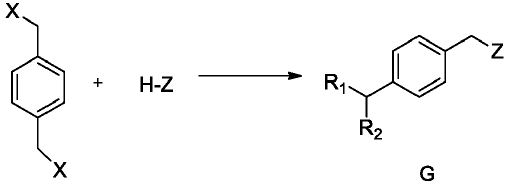

Wherein X is halogen, L₁ is a suitable leaving group selected from halogen, OH, OMs, OTs, OTf and the like; L₂ is a suitable leaving group selected from halogen, CN, OH, -CH₂OH, -CHO, CH₃NO₂, ester group, -NR₄R₅, OTf, OTs, OMs, -C=CR₆, -C≡CR₇ and the like, wherein R₄, R₅, R₆, R₇ are short chain alkyl groups; Z is cyclopentanone group and its precursor form selected from 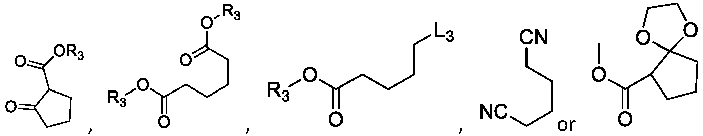 and the like; R₃ is short chain alkyl groups. L₃ is a suitable leaving group selected from halogen , OH, OMs, OTs, OT_{f} and the like, or organometallic groups. The invention also include the detailed procedure to convert the precursor compounds of cyclopentanone group to cyclopentanone, followed by the transformation of the precursor compounds of loxoprofen to loxoprofen.

## Description

This application claims priority of Chinese patent application submitted to the Chinese Patent Office on September 7, 2017, with the application number 201710800788.8, and entitled "Preparation Method For Substituted Phenylacetic Acid Derivatives". All of its contents are incorporated in this application by reference.

### Field of the invention

The invention belongs to the field of pharmaceutical manufacturingin reference to a preparation method of substituted phenylacetic acid derivatives, specifically relates to 2-(4-((2-oxocyclopentyl)methyl)phenyl)propanoic acid.

### Background of the invention

Substituted phenylacetic acid derivatives are disclosed in US patents US4161538, with good pharmaceutical activity of anti-inflammatory, analgesic and antipyretic. the structure is shown as follows:

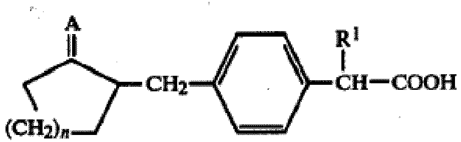

When A is oxygen and n=1 meanwhile and R¹ is methyl group,in the above general formula structure, the representative substituted phenylacetic acid is loxoprofen. The structure is shown as follows:

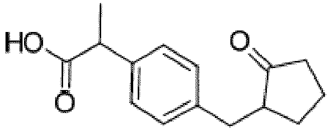

Loxoprofen is a non-steroidal anti-inflammatory type drug of with propionic acid moiety . The propionic acid derivatives drug family also include ibuprofen and naproxen et al. The loxoprofen has been launced in Brazil, Mexico and Japan in the form of sodium salt wherein hornored by Sankyo. The trade names of the loxoprofen sodium in Japan, Argentina and India are Loxonin, Oxeno and Loxomac respectively. Loxoprofen sodium is used for oral administration in these countries, and the injection administration form is approved to sell in Japan in January 2006.

In the patent US4161538, it disclosed the following synthetic route to prepare loxoprofen, wherein n=1, R¹ represents methyl group.

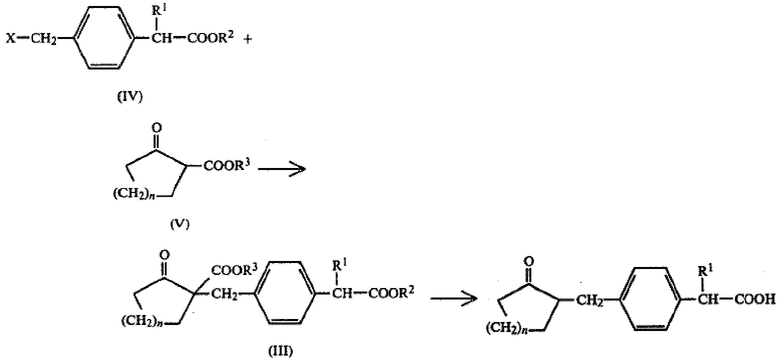

In the patent US5681979, it disclosed the compound with the following formula:

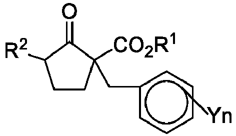

However, this formula was not used for the preparation of loxoprofen in this patent.

Owing to the good medical prospects of loxoprofen sodium, it is necessary to develop more excellent process for the preparation of loxoprofen.

### Summary of the invention

The present invention provides a synthetic process of substituted phenylacetic acid derivatives including loxoprofen. The novel process can synthesize the substituted phenylacetic acid and its derivaties in low-cost and high yield.

Firstly, the present invention provides an intermediate compound of general formula G and G₁: 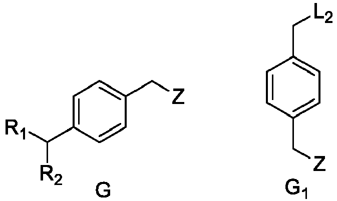 wherein R₁ is hydrogen or short chain alkyl groups; R₂ is halogen, CN, OH, -CH₂OH, -CHO, CH₃NO₂, ester group, -NR₄R₅, OTf, OTs, OMs, -C=CR₆, or -C≡CR₇, wherein R₄, R₅, R₆, R₇ are short chain alkyl groups; Z is cyclopentanone group and its precursor form selected from 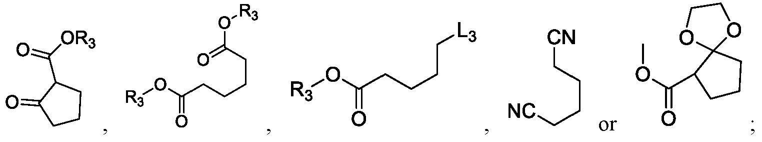 R₃ is short chain alkyl groups; The definition of L₂ is same with R₂. L₃ is a suitable leaving group selected from halogen , OH, OMs, OTs, OT_{f} and the like, or organometallic groups.

Secondly, The invention further provides a method for preparing general formula G and G₁ compounds by the reaction of 1,4-di-halobenzyl compounds or disubstituted benzyl compounds with the precursor form of cyclopentanone group. The reaction scheme is shown as follows : 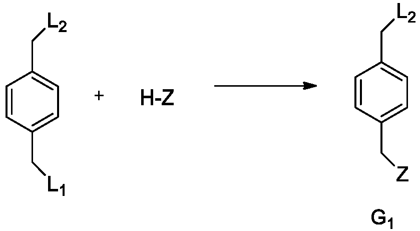 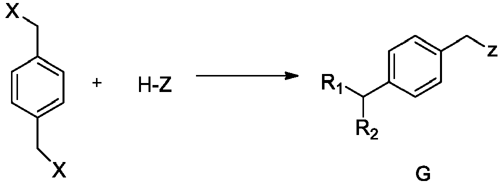 Wherein X is halogen, L₁ is a suitable leaving group selected from halogen, OH, OMs, OTs, OTf and the like; L₂ is same with the definition of R₂; Z represents cyclopentanone group and the precursor form of cyclopentanone group, the precursor form is 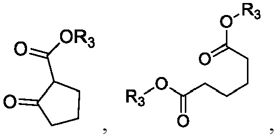 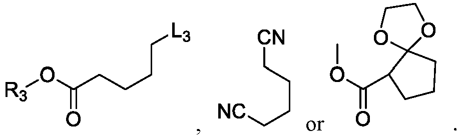 L₃ is a suitable leaving group selected from halogen , OH, OMs, OTs, OT_{f} and the like, or organometallic groups.
The preparation of above disubstituted benzyl compounds is carried out starting from 1,4-di-halobenzylcompounds or 1,4-dihydroxylbenzyl alcohol. The reaction scheme is shown as follows : 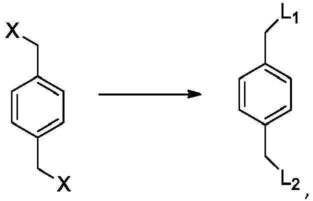 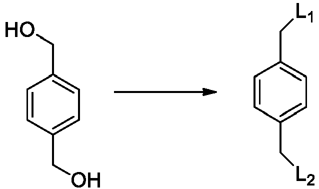 Wherein X is halogen, R₃ is short chain alkyl groups, L₁ or L₂ is a suitable leaving group selected from OMs, OTs, OTf, CH₃NO₂, -CN, -C≡C or 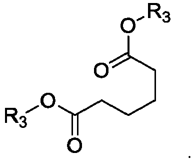 When L₂ is 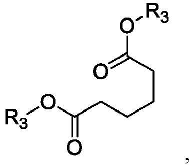 the reaction is shown as follows : 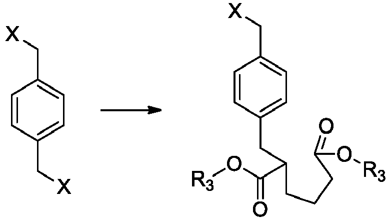 Wherein X is halogen, R₃ is short chain alkyl groups.

The above formula G and formula G₁ for the preparation of loxoprofen include the decarboxylation step.

The sequence of the decarboxylation step can be the first step, the second step or the third step, which means that the sequence is changeable.And it is the best sequence that the decarboxylation ahead of the cyanation step.

The above formula G and formula G1 for the preparation of loxoprofen include the step of transforming the precursor form of cyclopentanone group to cyclopentanone. The sequence of the transformation step can be the first step, the second step or the third step.

The invention further provides compound of formula III, the structure is shown as follows :

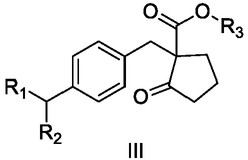

Wherein R₁ is H or short chain alkyl groups; R₂ is the group selected from halogen, -CN, -OH, -CH₂OH, -CHO, CH₃NO₂, ester groups, -NR₄R₅, OTf, OTs, OMs, -C=CR₆, -C≡CR₇ and the like, wherein R₄, R₅, R₆, R₇ is alkyl groups; R₃ is short chain alkyl groups.

In above formula III, when R₁ is H, R₂ is halogen, the formula of the compound is shown as follows: 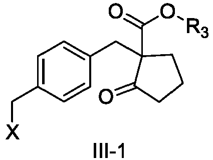 The definition of R₃ is the same as above.

The above compound of formula III-1 is prepared by the reaction of 1,4-bis(halomethyl)benzene and alkyl 2-oxocyclopentanecarboxylate, the reaction is shown as follows: 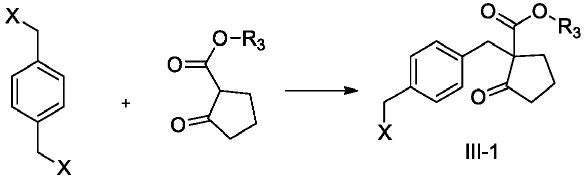 Wherein X is halogen, R₁ is H, the definition of R₃ is the same as above.

In above formula III, when R₁ is H, R₂ is OH, the formula of the compound is shown as follows: 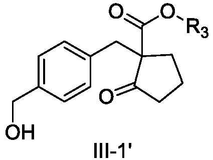 The above compound of formula III-1' is prepared by the reaction of 1,4- phenylenedimethanol and alkyl 2-oxocyclopentanecarboxylate, the reaction is shown as follows: 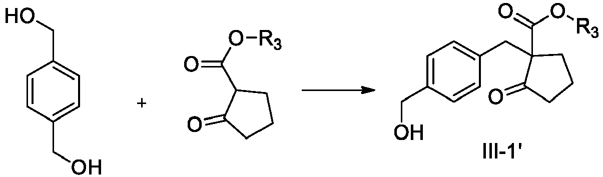 The definition of R₃ is the same as above..

In the above formula III, R₂ can be prepared from compound of formula III-1 and compound of formula III-1'. Alternatively, treatment of 1,4- phenylenedimethanol or 1,4-bis(halomethyl)benzene with sulfonation, amination or homocoupling reaction, then proceeding substitution. Or it can be prepared by Grignard reaction, and further react with carbon dioxide. The sulfonation reaction is shown as follows: 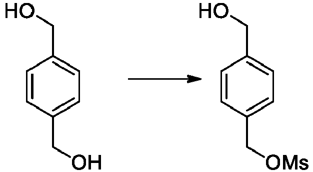 In above formula III, when R₂ is cyano group, the formula of the compound is shown as follows: 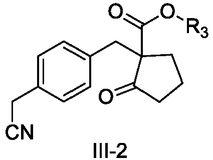 Wherein the definition of R₃ is the same as above.
The above compound of formula III-2 is prepared by the cyanation reaction of formula III-1, the reaction is shown as follows: 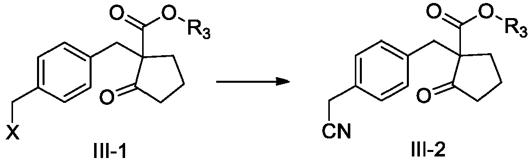 Wherein the definition of R₃ is the same as above.

In above formula III, when R₁ is short chain alkyl group, the formula of the compound is shown as follows: 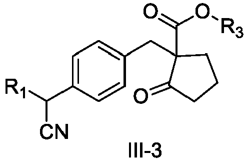 Wherein the definition of R₃ is the same as above.

The above compound of formula III-3 is prepared by alkylation of formula III-2, the reaction is shown as follows: 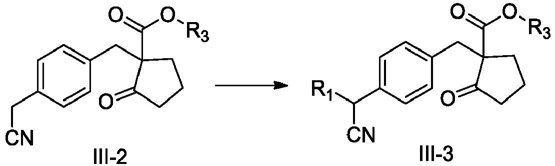 Wherein the definition of R₁ and R₃ are the same as above.

In addition, the above three steps such as ① substitution reaction of alkyl 2-oxocyclopentanecarboxylate, ② cyanation reaction, ③ alkylation reaction, the reaction sequence can adopt in any sequence. For example, the sequence can be ①②③, ①③②, ③②①, ②①③ or ②③①.

When the reaction sequence is ③②①, the reaction is shown as follows:

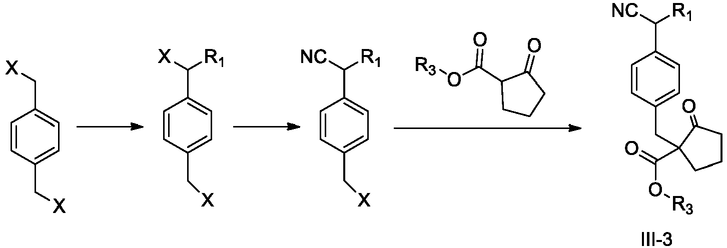

Wherein X is halogen, the definition of R₁ and R₃ are the same as above.
When the reaction sequence is ②③①, the reaction is shown as follows: 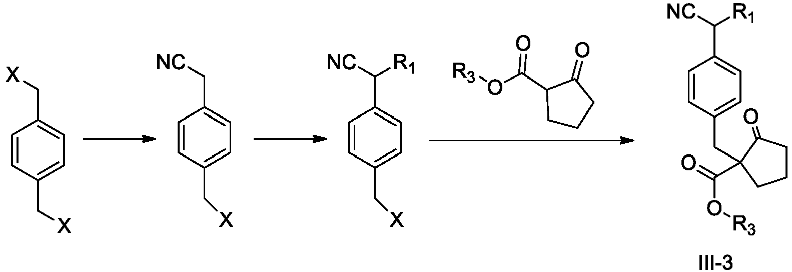 Wherein X is halogen, the definition of R₁ and R₃ are the same as above.

In the present invention, the substitution of alkyl 2-oxocyclopentanecarboxylate is carried out under base condition, the base is potassium carbonate, sodium carbonate, sodium alkoxide and the like.

In the present invention, the cyanation reaction is proceeded by using common cyanating reagent, such as NaCN, KCN, CuCN₂ and the like.

In the present invention, the alkylation reaction is proceeded by using common alkylating reagent, such as dimethyl carbonate, dimethyl sulfate, trimethoxymethane, alkyl halide and the like.

The above reaction in the invention can be carried out under the action of the organic solvent. The organic solvent may be the solvent commonly used in substitution, alkylation and cyanation reaction. The organic solvent include DMF, DMSO, NMP, 1,4-dioxane, methanol, ethanol, ethyl acetate, THF, MTBE, and acetonitrile et al.

The intermediate compound of this invention is used for preparing loxoprofen. It is best that the compound of formula III-3 can be hydrolyzed to produce related product, the reaction is shown as follows: 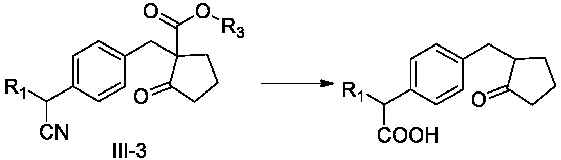 The definition of R₁ and R₃ are the same as above, when R₁ is methyl, the structure is loxoprofen.

The hydrolytic reagent uses for the hydrolysis reaction is an acid commonly used in this field, which can be an organic or inorganic acid, such as sulfuric acid, hydrochloric acid or trifluoroacetic acid.

The invention uses 1,4-bis(halomethyl)benzene compound as the starting material, and through substitution reaction, cyanation reaction and alkylation reaction in any order to produce the intermediate of formula III,

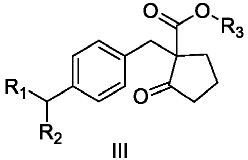

Wherein R₁ is H or short chain alkyl groups, R₂ is halogen or cyano group, R₃ is low-substituted alkyl groups.

When R₁ is methyl, R₂ is cyano group, R₃ is short chain alkyl groups, the compound of formula III-3 can be hydrolyzed to produce loxoprofen.

The invention discloses a preparation method for loxoprofen-like compounds, including a step for conversion of a cyclopentanone group in the form of a precursor to cyclopentanone. For example, the commonly used cyclization reaction in this field is used. The precursor compound of loxoprofen is used to prepare loxoprofen compounds.

On the other hand, the invention also provides a preparation method of loxoprofen compounds prepared by substitution reaction, decarboxylation reaction, cyanation reaction and alkylation reaction of 14-bishalomethlbenzene the reaction is shown as follows:

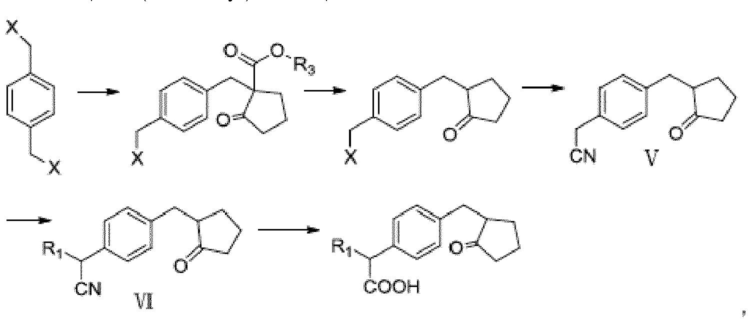

Wherein X is halogen, the definition of R₁ and R₃ are the same as above.

The preparation method provided by the invention has the following beneficial effects. Firstly, it provides an alternative method for preparing derivatives of substituted phenylacetic acid. Secondly, the preparation method of the invention is not reported by any prior arts. It is completely different from the compound used in the patent US5681979. Thirdly, In the reaction process, using 1,4-bis(halomethyl)benzene as starting material is cheap and convenient. At last, The preparation method provided by the invention is suitable for industrial scale production and has certain economic benefits.

### Detailed Description

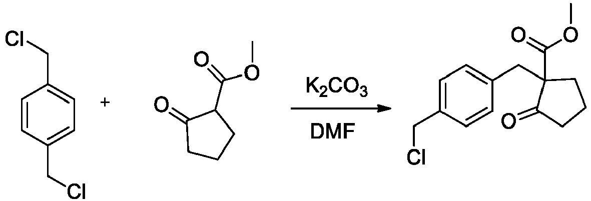

### Example 1:

1,4-bis(chloromethyl)benzene (30 g, 0.17mol), DMF (150g, 4.74vol) and sodium carbonate (19.8g, 0.19mol) were added to a 250 mL round bottom flask. The reaction mixture was stirred and heated to 60 °C. Then methyl 2-oxocyclopentanecarboxylate (22.1g, 0.16mol) was added to the reaction mixture dropwise in one hour, afterwards maintain the reaction temperature for 30 minutes. Then the reaction mixture was cooled to 25 °C, filtrate and mixe the mother liquor with water. The solid is separated. The mother liquor was extracted with EtOAc. Concentrate the organic phase to give the compound of Formula 111-1(47.0 g) with a yield of 83.3% (HPLC purity of 83.3%) (wherein R₁ is H, R₂ is Cl, R₃ is methyl).

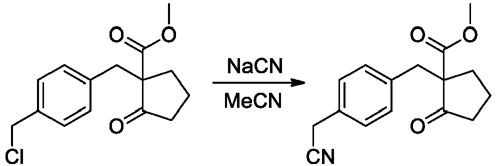

### Example 2:

The compound of Formula III-1 (wherein R₁ is H, R₂ is Cl, R₃ is methyl) (10g, 0.036mol), acetonitrile(50g, 5 vol), NaCN (1.9g, 0.039 mol) were added to a 100 mL round bottom flask. The reaction mixture was refluxed. When starting material disappeared, the reaction mixture was cooled to 25 °C. After filtration and evaporation, adding EtOAc and water while stirring. The organic layer was separated and evaporated to give the compound of Formula III-2 (9.8g) with the yield of 94.7% (HPLC purity of 93.7%) (wherein R₁ is H, R₂ is CN, R₃ is methyl).

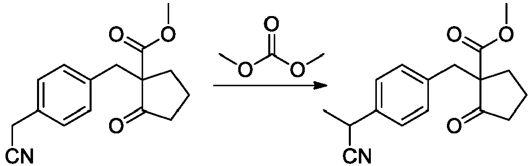

### Example 3:

The compound of Formula III-2 (wherein R₁ is H, R₂ is CN, R₃ is methyl) (30 g, 0.11 mol), dimethyl carbonate (24.8 g, 0.28 mol), K₂CO₃ (1.5 g, 0.011 mol), and tetrabutylammonium bromide (1.8 g, 0.006 mol) were added to an autoclave. The reaction mixture was stirred under 130-140 °C for 10h. The pressure of the autoclave is approximately 0.3Mpa. Then the reaction mixture was cooled to 28 °C, quenched by adding small amount of benzaldehyde. Flitration and the filter cake was washed by EtOAc, 1 N HCl and water. The organic layer was separated and evaporated to give the compound of Formula III-3 (32.6g) with the yield of 80.5% (HPLC purity of 78.1%) (wherein R₁ is methyl, R₂ is CN, R₃ is methyl).

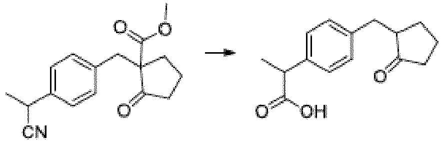

### Example 4:

The compound of Formula III-3 (wherein R₁ is methyl, R₂ is CN, R₃ is methyl) (18.5 g, 0.065 mol) and H₂SO₄ (80 wt.% solution in water, 16g, 0.13 mol) were added to a 100 mL round bottom flask. The reaction mixture was stirred under 80-90 °C for 5h. When the starting material disappeared, the reaction mixture was cooled to 25 °C, adding EtOAc to extract. The organic layer was washed by water and evaporated to give loxoprofen with a yield of 96.2% (HPLC purity of 93.7%).

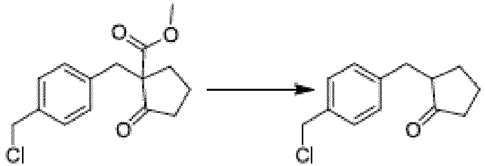

### Example 5:

The compound of Formula III-1 (wherein X is Cl, R₃ is methyl) (21 g, 0.075 mol), AcOH (37 mL, 2 vol) and HCl (35 wt.% solution in water, 63 mL, 3 vol) were added to a 100 mL round bottom flask. The reaction mixture was stirred under 90-95 °C for 2.5-3.5h. When starting material disappeared, the reaction mixture was cooled to 15-25 °C afterwards added water and EtOAc. The organic layer was washed with 5% NaHCO₃ solution, saturated NaCl solution and water. The organic layer was separated and evaporated to give the compound of Formula IV (19.7g) with the yield of 96.1%(HPLC purity of 81.4%) (wherein X is Cl).

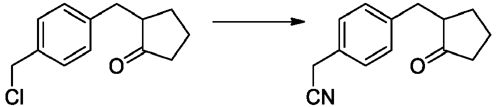

### Example 6:

The compound of of Formula IV (wherein X is Cl) (10g, 0.045mol), acetonitrile (50g, 5 vol), NaCN (2.4g, 0.049 mol) were added to a 100 mL round bottom flask. The reaction mixture was refluxed. When starting material disappeared, the reaction mixture was cooled to 25 °C. After filtration and evaporation, adding EtOAc and water to stir. The organic layer was washed by saturated NaCl solution and water; Then the organic layer was separated and evaporated to give the compound of Formula V (9.68g) with the yield of 94.7% (HPLC purity of 93.7%).

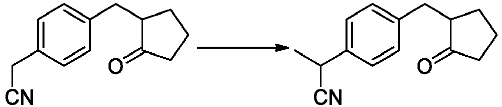

### Example 7:

The compound of Formula V (30 g, 0.14 mol), dimethyl carbonate (31.5 g, 0.35 mol), K₂CO₃ (1.5 g, 0.011 mol), and tetrabutylammonium bromide (1.8 g, 0.006 mol) were added to a 100 mL autoclave. The reaction mixture was stirred under 130-140 °C for 10h. The pressure of autoclave is approximately 0.3 Mpa. Then the reaction mixture was cooled to 28 °C, and quenched by adding small amount of benzaldehyde. Filtration and the filter cake was washed by f EtOAc, 1 N HCl and water. The organic layer was separated and evaporated to give the compound of Formula VI (31.7g) with a yield of 80.4% (HPLC purity of 81.1%) (wherein R₁ is H).

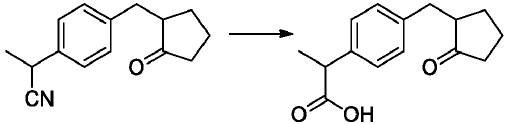

### Example 8:

The compound of Formula VI (wherein R₁ H) (18.5 g, 0.081 mol) and AcOH (37 mL, 2 vol) and HCl (35 wt.% solution in water, 55.5 mL, 3 vol) were added to a 100 mL round bottom flask. The reaction mixture was stirred under 90-95 °C for 2.5-3.5h. When starting material was disappeared, the reaction mixture was cooled to 15-25°C and was added water and EtOAc. The organic layer was washed with saturated NaCl solution and water; The organic layer was separated and evaporated to give loxoprofen (20.5 g) with the yield of 96.2% (HPLC purity of 93.7%).

## Claims

1. A compounds of formula G and formula G₁: 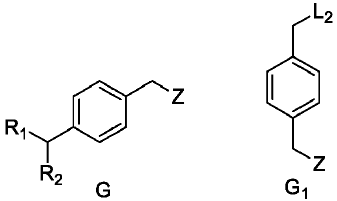 wherein R₁ is hydrogen or short chain alkyl groups; R₂ is halogen, CN, OH, -CH₂OH, -CHO, CH₃NO₂, ester group, -NR₄R₅, OTf, OTs, OMs, -C=CR₆, or -C≡CR₇, wherein R₄, R₅, R₆, R₇ are short chain alkyl groups; Z is cyclopentanone group and its precursor form selected from 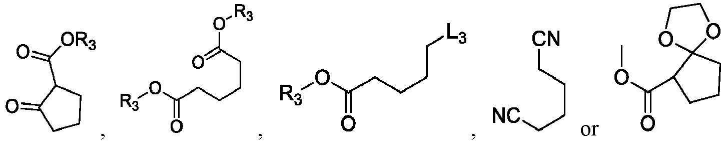 ; R₃ is short chain alkyl groups; The definition of L₂ is same with R₂. L₃ is a suitable leaving group selected from halogen , OH, OMs, OTs, OT_{f} and the like, or organometallic groups.

2. A compound of formula III: 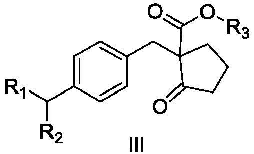 wherein R₁ is hydrogen or short chain alkyl groups; R₂ is the group selected from halogen, CN, OH, -CH₂OH, -CHO, CH₃NO₂, ester group, -NR₄R₅, OTf, OTs, OMs, -C=CR₆, -C≡CR₇ and the like, wherein R₄, R₅, R₆, R₇ are short chain alkyl groups; R₃ is short chain alkyl groups.

3. The compound refer to claim 2 with the the formula as III-1, III-2 and III-3: 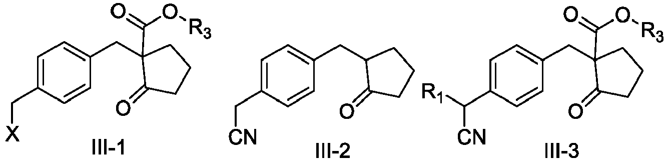 Wherein X is halogen; R₁ and R₃ is short chain alkyl groups.

4. A preparation method of formula G and formula G1 refer to claim 1. Wherein by the reaction of 1,4-di-halobenzylcompounds or disubstituted benzyl compounds with the precursor of cyclopentanone group. The reaction scheme is shown as follows: 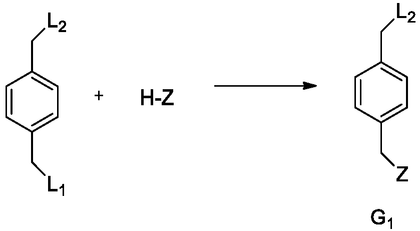 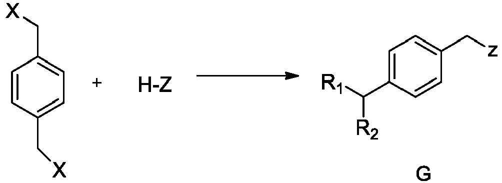 Wherein X is halogen, L₁ is halogen, OH, OMs, OTs, OTf; L₂ is same with the definition of R₂ in Claim 1; The definition of Z is same with above in Claim 1.

5. The preparing method refer to Claim 4. Wherein the disubstituted benzyl compounds is prepared from 1,4-di-halobenzyl compounds or 1,4-dihydroxylbenzyl alcohol. The reaction scheme is shown as follows: 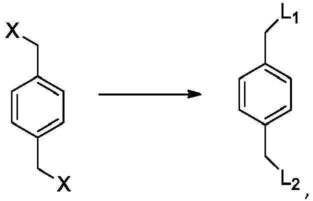 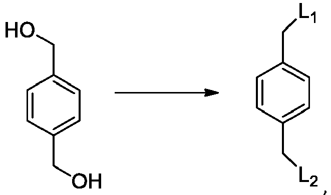 Wherein X is halogen, R₃ is short chain alkyl groups, L₁ or L₂ is OMs, OTs, OTf, CH₃NO₂, - CN, -C≡C or

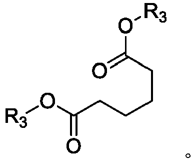

6. Formula G and formula G₁ of claim 1. Wherein the preparation method of loxoprofen include the decarboxylation step.

7. According to claim 6, the sequence of decarboxylation is from the first step to the second step and the third step.

8. A preparation method of formula III-1 of claim 3. Wherein the reaction between the 1,4-bishalobenzyl compounds and alkyl 2-oxocyclopentanecarboxylate. The reaction scheme is shown as follows: 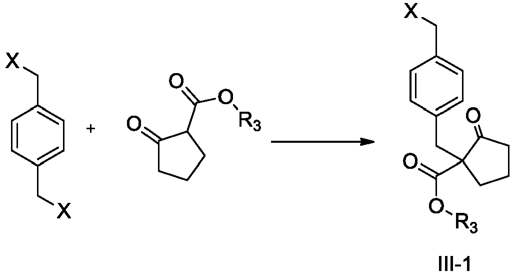 Wherein X is halogen, R₃ is short chain alkyl groups.

9. A preparation method of formula III-2 of claim 3 is prepared by cyanation reaction with formula III-1. The reaction scheme is shown as follows: 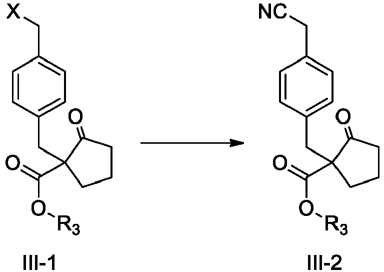 Wherein X is halogen, R₃ is short chain alkyl groups.

10. A preparation method of formula III-3 of claim 3 is prepared by alkylation of formula III-2. The reaction scheme is shown as follows: 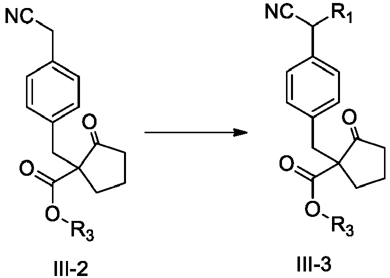 Wherein R₁ and R₃ are short chain alkyl groups.

11. A preparation method of formula III-3 of claim 3. Wherein formula III-3 is prepared by the substitution, cyanation and alkylation reaction in any order. The reaction scheme is shown as follows: 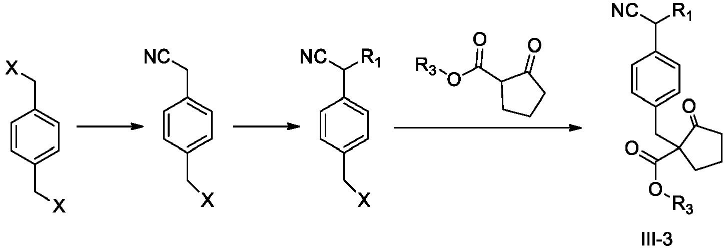 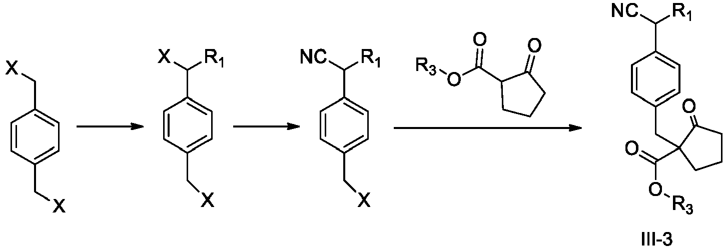 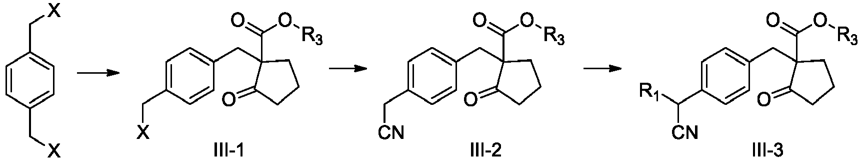 Wherein X is halogen, R₁ and R₃ are short chain alkyl groups.

12. Role of formula III-3 in claim 3. Wherein loxoprofen and its derivatives are prepared via decaboxylation reaction and hydroxylation reaction . The reaction scheme is shown as follows: 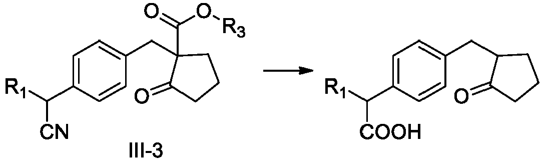 Wherein R₁ is short chain alkyl groups.

13. Role of formula G and formula G₁ in claim 1. Wherein the loxoprofen or its derivatives are prepared by the conversation of precursor form of cyclopentanone group to cyclopentanone.

14. A method for preparing loxoprofen or its derivatives can be proceeded by substitution, decarboxylation, cyanation, alkylation and hydroxylation reactions. The reaction scheme is shown as follows: 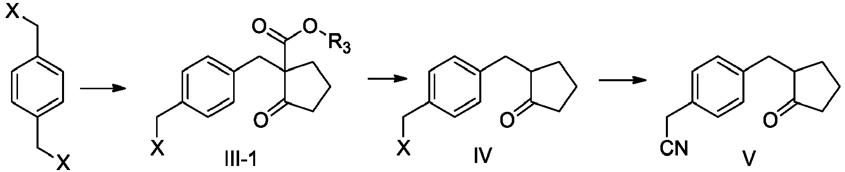 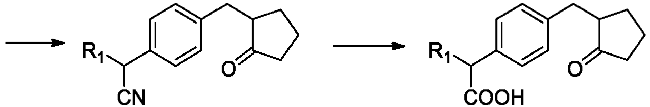 Wherein X is halogen, R₁ and R₃ are short chain alkyl groups.
